# EUROPEAN PATENT SPECIFICATION

(11) **EP 3 681 416 B1**
(45) Date of publication and mention of the grant of the patent: **05.11.2025**
(21) Application number: 18778364.2
(22) Date of filing: 12.09.2018
(51) Int. Cl.: A61B 17/17, A61B 17/88, A61B 17/56

(54) **PATIENT-SPECIFIC TEMPLATES FOR PEDICAL SCREW INSERTION IN CORRECTIVE SCOLIOSIS SURGERIES**
PATIENTENSPEZIFISCHE SCHABLONEN FÜR DIE INSERTION VON PEDIKELSCHRAUBEN IN DER CHIRURGIE ZUR KORREKTUR VON SKOLIOSE
MODÈLES SPÉCIFIQUES AU PATIENT POUR UNE INSERTION DE VIS PÉDICULAIRE DANS DES CHIRURGIES CORRECTIVES DE SCOLIOSE

(30) Priority: 14.09.2017 EG 2017090025; 13.03.2018 EG 2018030448
(43) Date of publication of application: 22.07.2020
(73) Proprietor: Hafez, Mahmoud Alm El Din, Giza (EG)
(72) Inventor: AL OTHMAN, Abdullah Ahmed, Alkhober East Area (SA); HAFEZ, Mahmoud Alm El Din, Giza (EG)
(74) Representative: Mathys & Squire
(86) International application number: PCT/EG2018/000014
(87) International publication number: WO 2019/052623

(56) References cited:
- WO-A1-2016/075581
- US-A1- 2014 350 614

## Description

### Technical Field

The present invention relates to a device for determining the position, the path, and the size of pedicle screws used in corrective scoliosis surgeries. The device is a patient-specific template with data about the size, path, and position of the screw.

### Prior Art

### Disadvantages of Current Technologies

The increasing use of pedicle screws in corrective scoliosis surgeries has posed a challenge to surgeons, especially those of limited experience. This kind of surgery is critical and should be conducted by professional surgeons. The major issue in corrective scoliosis surgery is determining the right position and path of the pedicle screw in the vertebra. Mislocating the pedicle screw leads to unsatisfactory results for both the surgeon and the patient. For example, if the screw gets out of its path it may cause compression of the nerves connected to the spine, difficulty of leg motion after surgery or loss of contact with the aorta artery behind the spine. According to previous studies and researches, more than 25% of pedicle screws are mislocated in corrective scoliosis surgeries. Examples for prior art solutions can be found in US 2014/350614 and WO 2016/075581. Furthermore, a most notable document is US 2014/350614 A1. Which offers better seating in terms of utilizing contact on transverse process as well as more surface contact on the spinous process as well as simultaneous seating on several vertebra at once on one of its embodiments. However, the latter configuration that utilize seating on several vertebra at once isn't guaranteed given the different relative position of the vertebrae to each other during supine position in scanning and medical imaging and prone position during surgery.

Accordingly, there is a dire need for a new device that uses guides and patient-specific electronic templates for accurately determining the path, location, and size of the pedicle screw for each vertebra

### Summary of the invention

The invention is defined in the attached claims.

The invented device has zero error ratio, as the template is designed to match the patient's vertebrae topology.

### Description of the Figures

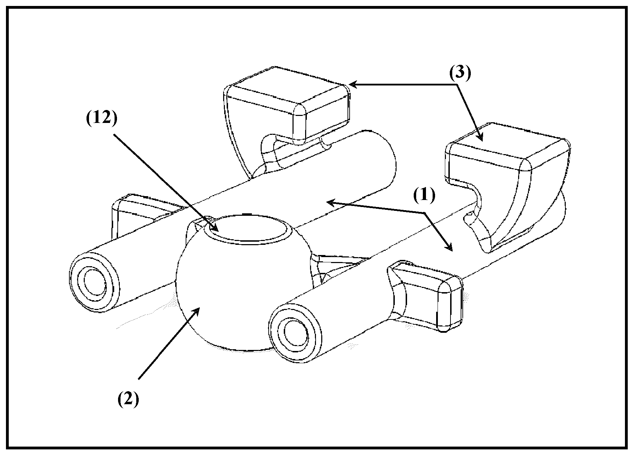

Figure no. 1: represents a three-dimensional perspective for the patient-specific electronic template used for determining the position, the path, and the size ofthe pedicle screw. The template appears with two hollow cylinders (1), each ends with a flank (3). It also includes a central sphere at its center (2) with two flat surfaces (12).

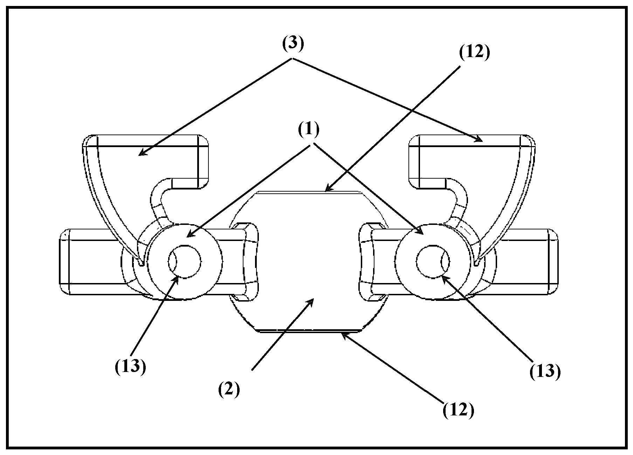

Figure no. 2: represents a two-dimensional front view for the patient-specific electronic template for determining the position, the path, and the size of the pedicle screw. The template appears with two hollow cylinders (1), each has a central hole with fixed diameter . Both hollow cylinders ends with a flank (3). It also includes a central sphere at its center (2) with two flat surfaces (12).

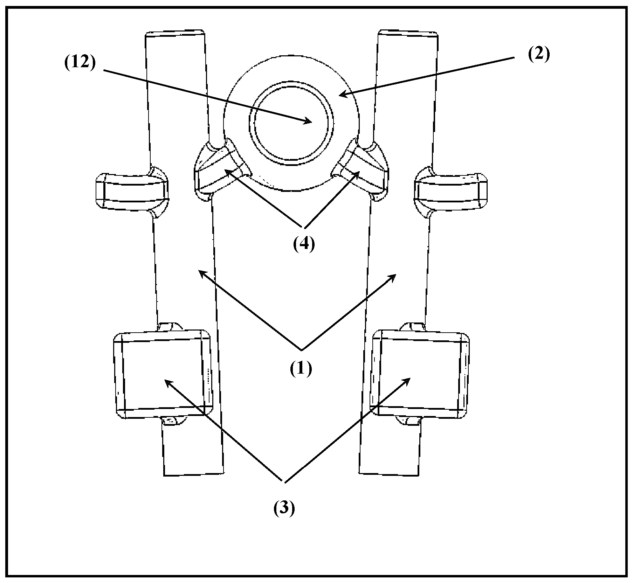

Figure no. 3: represents a two-dimensional plan view for the patient-specific electronic template for determining the position, the path, and the size of the pedicle screw. The template appears with two hollow cylinders (1), each ends with a flank (3). It also includes a central Sphere (2) at its center . The connectors (4) appear between the central sphere (2) and the hollow cylinders (1). The central sphere (2) has two flat surfaces (12).

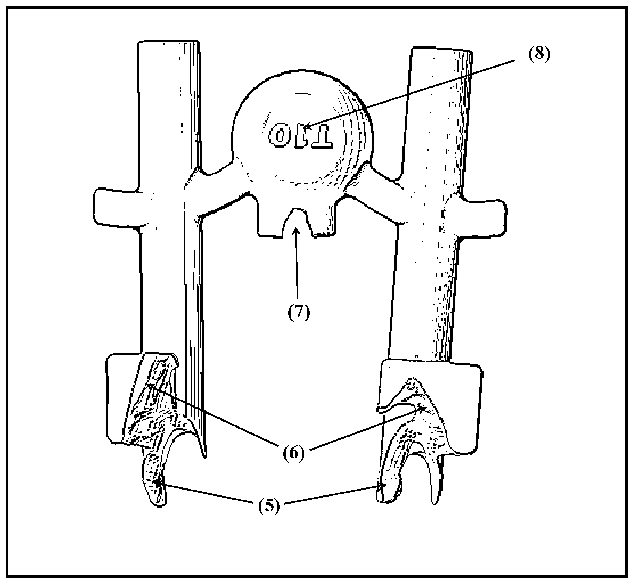

Figure no. 4: represents a two-dimensional plan view for the patient-specific electronic template for determining position, the path, and the size of pedicle screw after the planning process. The end of the hollow cylinders' surface (5) appears with a topology that matches that of the plate surface and the transverse process of the targeted vertebra. The flanks at the end of each cylinder (6) have the same topology. The central sphere has a cavity (7) that fits the outer surface of the spinal process . The number of the vertebra to be provided with the template is registered on the flanks to avoid confusion during surgery (8).

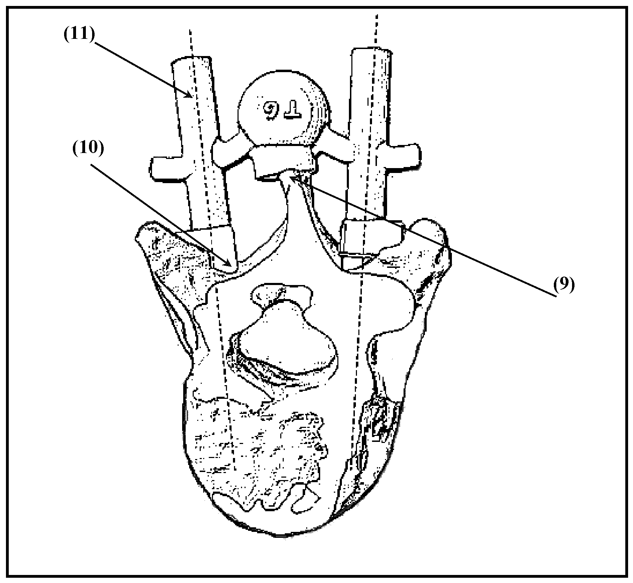

Figure no. 5: represents a two-dimensional plan view for the patient-specific electronic template for determining the position, the path, and the size of pedicle screw fixed on the targeted template. The figure illustrates the location of the cylinders' ends, the plate's flanks, the transverse process (10), and the location of the centre sphere's cavity (7) on the spinal process (9). A centerline for the axis for the pedicle screw path inside the template and the vertebra (11) appears in the figure.

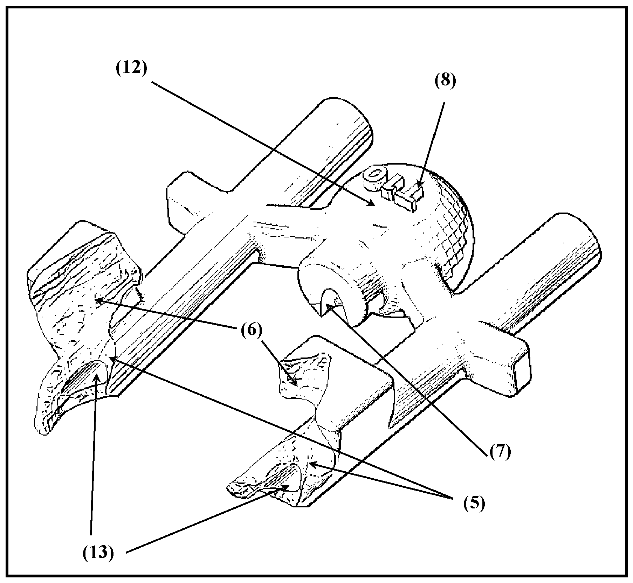

Figure no. 6: represents a three-dimensional perspective for the patient-specific electronic template for determining the position, the path, and the size of pedicle screw after the planning process. The end of the hollow cylinders' surface (5) with central hole (13) appears with a topology that matches that of the plate surface and the transverse process of the targeted vertebra. The flanks at the end of each cylinder (6) have the same topology. The central sphere has a cavity (7) that fits the outer surface of the spinous process . The number of the vertebra (8) is to be provided with the template on flat surfaces (12).

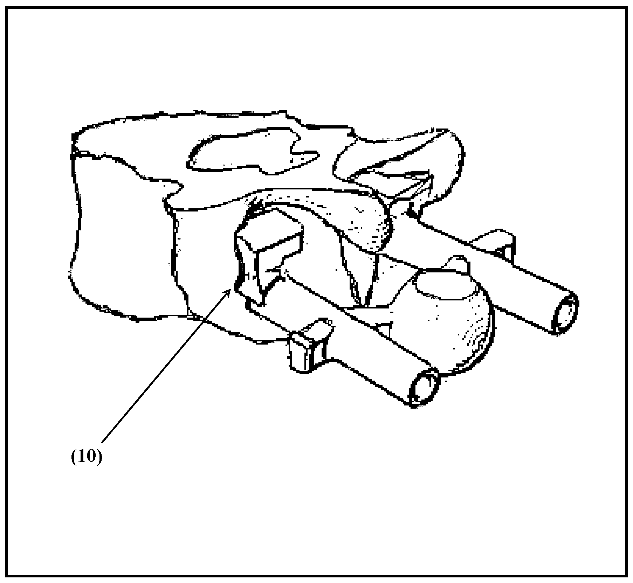

Figure no. 7: represents a three-dimensional perspective for the patient-specific electronic template for determining the position, the path, and the size of pedicle screw to be fixed on the targeted vertebra. The figure illustrates the location of the cylinders' ends, the plate's flanks and the transverse process (10).

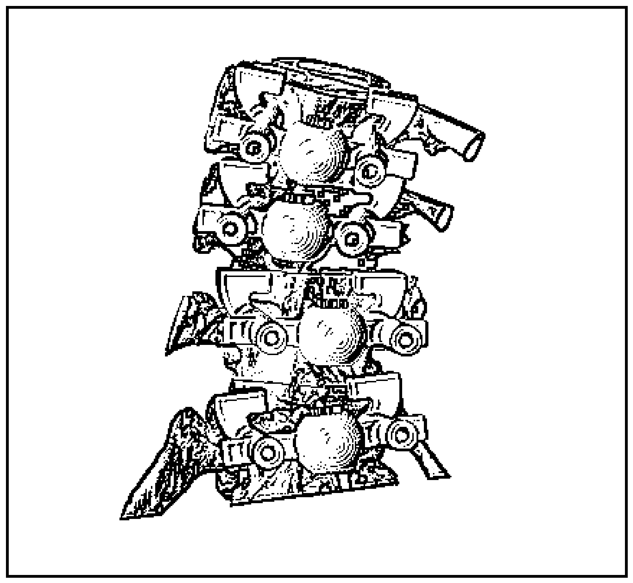

Figure no. 8: represents a two-dimensional front view for a number of electronic templates fixed on their proper positions on the targeted vertebra.

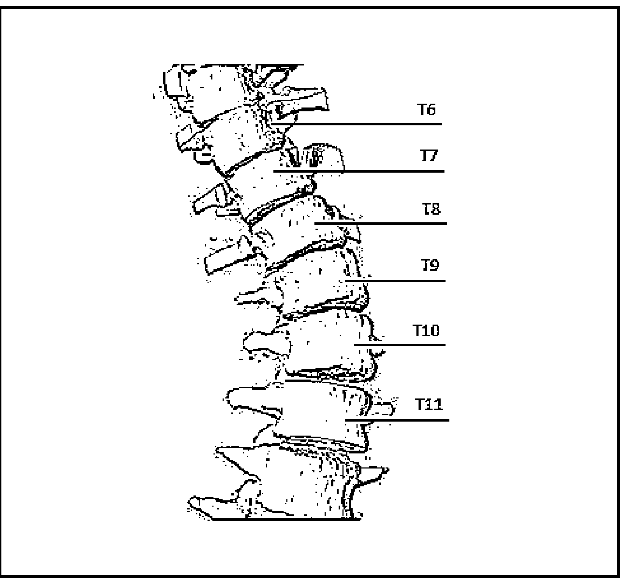

Figure no. 9: represents a front view of the spine in which some vertebrae appear with conventional numbers that is registered on the template for avoiding confusion during surgery.

### Detailed Description of the Invention

The current invention relates to a device for determining the position, the path, and the size of pedicle screws used in corrective scoliosis and spinal disc herniation surgeries. The device is a patient-specific template used for single patient only with data about the size, path and position of the screw. The template is designed according to anatomical indicators and markers of the spinal vertebrae. The template includes two hollow cylinders (1) with flanks (3) for vertebrae matching and template fixation. Italso contains a central hollow sphere (2) that matches the topology of the spinous process (9) of the vertebrae on which the template would be fixed. The said sphere (2) is connected to the cylinders by two connectors (4) (Figures no. 1, 2, 3, 4 & 6).

The electronic template is manufactured according to a pre-operative planning using a special software program.

The surgery is planned according to the program's data input. The patient undergoes a computed tomography scan (CT scan) that is converted to three-dimensional scan of the patient's spine. Each vertebra appears independent from the other, hence ensuring accurate surgery planning and correct determination of the position and path of the pedicle screw for each vertebra.

Pre-surgery planning is made depending on the anatomic form of the vertebra, putting into consideration the shape of the spinous process, the internal plate and the transverse process as anatomic indicators for the fixation of the electronic template on the vertebra. In the planning process, the degree of the spine curvature is determined as well as the position of each vertebra, its degree of rotation and inclination relative to the vertebral column axis. In this way, the position, the path, the inclination angle and all data related to the pedicle screw are determined. The position of the electronic template on the vertebra is determined by the aid of this data as well as surface topologies of the spinous process, the transverse process and the plate that are matched to the outer surface of the hollow cylinders (5) and the flanks (6) at the cylinders' ends (5) (see figures 4, 5, 6 & 7).

The said electronic templates are fixed on the vertebra depending on its outer surface's topology, according to which the template's internal surface is formed at the end of the hollow Cylinders (5) and their flanks (6). Hence, the template is only fitted into one location on the vertebra's surface during the pre-planned surgery. This makes it easier for surgeons, especially oflimited experience, to accurately determine the position, the path and the size ofthe pedicle screw to be fixed on the vertebra. It is impossible for the template to be mislocated. It cannot be displaced since its unique design makes the surface ofthe hollow cylinder end identical to the
vertebra's surfaces on which it is to be fixed (see figure no. 5 & 7).

The template's design is based on the use of two hollow cylinders (1) (figure no. 1) through which the wire or surgical drill passes forming the void space of the pedicle screw in the vertebra.

There is an interfacial angle between the cylinders that varies according to the interfacial angle between the specific paths of the concerned pedicle screws (see figure no. 8). In detail, each vertebra has its own template that contains two hollow cylinders of fixed diameter. Each hollow cylinder has a central hole (13) with a fixed diameter, through which the wire or surgical drill passes through toward the two flanks (3) whose topology matches the surface of the vertebra. The interfacial angle between the cylinders differs from one template to another according to the vertebra's number that indicates its location, whether it is lumbar vertebra or thoracic vertebra (figure no. 9), and according to the path inclination of the pedicle screw as a main component of the invention (see figure no. 5).

As previously mentioned, the topology of the cylinders' ends (6) (the surface that contacts the vertebra's surface) matches that of the vertebra's surface (figure no. 4). The template has a central sphere (2) with a cavity (7) that matches the outer surface of the spinous process for fixing the template on its pre-designed location on the vertebra only (see figures no. 5 & 7). In other words, the surgeon cannot fix the template in a position other than that assigned during the computer-assisted surgical planning (see figures no. 5 & 7).

The template contains two flanks (3) at the end of each cylinder whose surface (5) matches the outer surface of the vertebral plate. They are used for keeping the template fixed to help the surgeon to determine the pedicle screw's position and path in the vertebra. The template includes two connectors (4) between the central sphere (2) and the hollow cylinders (1) to increase the space of the template and make it tolerant to the forces exerted by surgical instruments. In this way, the template is kept safe from breakage or displacement.

The template is produced through three-dimensional printing techniques.

## Claims

1. A patient-specific template for single-use in corrective surgeries addressing scoliosis and spinal disc herniation, comprising two hollow cylinders (1), each terminating with a surface (3) replicating the shape of the corresponding vertebra; a central sphere (2) with a cavity (7) that securely fits the upper surface of the spinal process and two middle connectors (4) between the central sphere and the hollow cylinders to enhance structural durability, wherein said sphere (2) is connected to the cylinders (1) by the two connectors (4).

2. The template according to claim 1, **characterized by** the two hollow cylinders (1) being configured to extend on the vertebra's surface, wherein each of the two hollow cylinders (1) is equipped with a flank (3) at its termination.

3. The template according to claim 1, **characterized in that** there is an interfacial angle between the two hollow cylinders (1) that is determined based on the intended inclination of the pedicle screws .

## Patentansprüche

1. Patientenspezifische Vorlage zur einmaligen Verwendung bei Korrekturoperationen zur Behandlung von Skoliose und Bandscheibenvorfall, mit zwei Hohlzylindern (1), die jeweils mit einer Oberfläche (3) enden, welche die Form des entsprechenden Wirbels nachbildet; einer zentralen Kugel (2) mit einem Hohlraum (7), der sicher auf die obere Fläche des Wirbelfortsatzes passt, und zwei mittleren Verbindern (4) zwischen der zentralen Kugel und den Hohlzylindern, um die strukturelle Haltbarkeit zu verbessern, wobei die Kugel (2) mit den Zylindern (1) durch die beiden Verbinder (4) verbunden ist.

2. Vorlage nach Anspruch 1, **dadurch gekennzeichnet, dass** die beiden Hohlzylinder (1) so konfiguriert sind, dass sie sich auf der Oberfläche des Wirbels erstrecken, wobei jeder der beiden Hohlzylinder (1) an seinem Ende mit einer Flanke (3) versehen ist.

3. Vorlage nach Anspruch 1, **dadurch gekennzeichnet, dass** zwischen den beiden Hohlzylindern (1) ein Grenzflächenwinkel besteht, der in Abhängigkeit von der vorgesehenen Neigung der Pedikelschrauben bestimmt wird.

## Revendications

1. Modèle spécifique à un patient destiné à un usage unique dans des chirurgies correctives portant sur la scoliose et la hernie discale spinale, comprenant deux cylindres creux (1), chacun se terminant par une surface (3) reproduisant la forme de la vertèbre correspondante ; une sphère centrale (2) avec une cavité (7) qui s'adapte solidement à la surface supérieure de l'apophyse épineuse et deux connecteurs médians (4) entre la sphère centrale et les cylindres creux pour améliorer la durabilité structurale, dans lequel ladite sphère (2) est connectée aux cylindres (1) par les deux connecteurs (4).

2. Modèle selon la revendication 1, **caractérisé par le fait que** les deux cylindres creux (1) sont configurés pour s'étendre sur la surface de la vertèbre, dans lequel chacun des deux cylindres creux (1) est équipé d'un flanc (3) à sa terminaison.

3. Modèle selon la revendication 1, **caractérisé en ce qu'**il y a un angle interfacial entre les deux cylindres creux (1) qui est déterminé sur la base de l'inclinaison voulue des vis pédiculaires.
